# FASCICULE DE BREVET EUROPEEN

(11) **EP 0 544 605 B1**
(45) Date de publication et mention de la délivrance du brevet: **14.05.1997**
(21) Numéro de dépôt: 92420436.5
(22) Date de dépôt: 24.11.1992
(51) Int. Cl.: C12Q 1/04, C12M 1/22

(54) **Milieu d'analyse microbiologique et procédé pour détecter des levures de l'espèce candida albicans**
Mikrobiologisches analytisches Milieu und Verfahren zum Nachweis von Hefen der Art Candida albicans
Microbiological analytical environment and procedure to detect yeasts of the species Candida albicans

(30) Priorité: 25.11.1991 FR 9114972
(43) Date de publication de la demande: 02.06.1993
(73) Titulaire: BIO MERIEUX, Société anonyme, F-69280 Marcy l'Etoile (FR)
(72) Inventeur: Orenga, Sylvain, F-69003 Lyon (FR)
(74) Mandataire: Guerre, Dominique

(56) Documents cités:
- US-A- 4 874 695
- DIAGNOSTIC MICROBIOLOGY AND INFECTIOUS DISEASE vol. 12, no. 6, 1989, NEW YORK NY USA pages 521 - 523 M.T. DALTON ET AL. 'Rapid identification of candida albicans using 4-methylumbelliferyl n-acetyl-beta-galactosaminide.'
- ABSTRACTS OF THE ANNUAL MEETING OF THE AMERICAN SOCIETY FOR MICROBIOLOGY vol. 77, 1977, WASHINGTON DC USA page 129 D.G. BOBEY ET AL. 'Detection of yeast enzymes using fluorescent substrates.'
- JOURNAL OF MICROBIOLOGICAL METHODS vol. 14, no. 2, 1 Août 1991, AMSTERDAM NL pages 103 - 108 M. MANAFI ET AL. 'Rapid identification of candida albicans by fluoroplate candida agar.'
- JOURNAL OF CLINICAL MICROBIOLOGY vol. 12, no. 6, 1989, WASHINGTON DC USA pages 2424 - 2425 J.L. PERRY ET AL. 'Umbelliferyl-labeled galactosaminide as an aid in identification of candida albicans.'

## Description

L'invention concerne l'identification des levures de l'espèce *Candida albicans*, par ensemencement d'un milieu de culture spécifique avec un échantillon présumé contenir lesdites levures, croissance de ces dernières sur le milieu de culture et développement sur ledit milieu d'un phénomène chromogène ou fluorigène de caractérisation, à partir d'un caractère biochimique spécifique de ladite espèce de levure.

Il existe en effet un caractère biochimique spécifique des *Candida albicans* consistant en une activité enzymatique exercée par un enzyme appartenant au groupe des hexosaminidases et présent dans l'espèce *Candida albicans.*

La détermination de cette activité enzymatique fait l'objet d'un test utilisant un milieu de culture et de détection, commercialisé par les Laboratoires MERCK-CLEVENOT sous la marque Fluoroplate Candida Agar, comprenant une base nutritive métabolisée par les levures, et un substrat fluorigène, susceptible d'être hydrolysé par la N-acétyl-β-D-galactosaminidase pour libérer un produit fluorescent. Le substrat fluorigène est la 4-méthylumbelliféryl-N-acétyl-β-D-galactosaminide ou 4-méthylumbelliféryl-2-acétamido-2-désoxy-β-D-galactopyranoside. L'hydrolyse enzymatique de ce dernier par *Candida albicans* libère la 4-méthylumbelliférone, un produit fluorescent bleu, détectable sous rayonnement UV à 360 nm.

Ce test a également été décrit dans le document suivant : M Manafi et al, Journal of Microbiological Methods, vol 14, n°2, Ier août 1991, Amsterdam NL, pages 103-108.

D'autres documents ont également décrit, avec une base nutritive appropriée, le même substrat fluorigène ou un substrat chromogène différent, susceptible d'être hydrolysé par un enzyme du type des hexosaminidases pour libérer un produit coloré ou fluorescent :
- selon le document US-A-4 874 695, on détecte une activité N-acétyl-β-D-galactosaminidase sur la p-nitrophényl-N-acétyl-β-D-galactosaminidase utilisée comme substrat,
- le résumé, Abstract of the Annual Meeting of the American Society for Microbiology, vol. 77, 1977, page 129, de D. G. BOBEY et al,
- JL Perry et al, Journal of Clinical Microbiology, vol 12, n°6, 1989, Washington DC, USA, pages 2424-2425, et
- MT Dalton et al, Diagnostic Microbiology and Infections Disease, vol 12, n°6, 1989, New-York, pages 521-523
ont décrit plus particulièrement l'utilisation de la 4-méthylumbelliferyl-N-acétyl-β-D-galactosaminide ou 4-méthylumbelliféryl-2-acétamido-2-désoxy-β-D-galactopyranoside.

Cependant, outre le fait que ces milieux de détection requièrent l'utilisation d'une source de rayonnement UV pour visualiser la réaction, cette dernière n'est que très faiblement détectable au bout de 24 heures et l'est faiblement au bout de 48 heures, rendant le test inutilisable dans le cadre d'une détection rapide.

De plus, la diffusion dans le milieu du produit fluorescent libéré par l'hydrolyse enzymatique, diminue la probabilité de mise en évidence de *Candida albicans* dans une culture plurimicrobienne.

Le problème de la mise au point d'un procédé pour détecter sélectivement et rapidement *Candida albicans* n'est donc pas encore résolu. Pourtant, le besoin est grand, étant donné que *Candida albicans* est l'espèce la plus communément isolée à partir d'échantillons cliniques, et provoque des infections plus ou moins importantes de la peau, des ongles et des muqueuses chez les individus présentant des défenses immunitaires normales et des infections très sérieuses chez les individus affaiblis et notamment ceux infectés par le virus HIV.

Selon la présente invention, un milieu de culture pour détecter *Candida albicans,* et un procédé le mettant en oeuvre, ont été élaborés permettant de pallier les inconvénients du test précité.

L'invention apporte également un dispositif de caractérisation microbiologique comprenant un conditionnement d'un milieu de culture et détection de *Candida albicans,* agencé pour permettre l'ensemencement d'un échantillon à tester sur le milieu et son incubation.

Le milieu de culture selon l'invention comprend la base nutritive, un substrat comprenant une partie hexosaminée liée par l'oxygène porté par le carbone anomère à un groupement chromogène ou fluorigène, et en outre, au moins un composé hexosaminé qui est différent du substrat.

Selon une mise en oeuvre particulière de l'invention, le composé hexosaminé appartient au groupe des hexosamines simples et, dans ce cas, le composé hexosaminé peut être identique ou différent de la partie hexosaminée du substrat.

Le composé hexosaminé est une molécule et la partie hexosaminée du substrat est un radical, aussi on comprendra leur identité à un atome d'hydrogène près.

S'agissant des hexosamines simples, le composé hexosaminé peut être une hexosamine N-substituée, et en particulier une N-acétyl-hexosamine ou 2-acétamido-2-désoxy-hexose.

Quand le composé hexosaminé est identique ou différent de la partie hexosaminée du substrat, on constate que contrairement à l'effet rétro-inhibiteur attendu, le composé se comporte dans le milieu de l'invention ensemencé par *Candida albicans*, comme un excellent activateur des hexosaminidases.

Selon une autre mise en oeuvre particulière de l'invention, le composé hexosaminé appartient au groupe des hexosamines complexes, et constitue un analogue structural du substrat, comprenant une partie hexosaminée, identique ou différente de la partie hexosaminée du substrat liée par l'oxygène porté par le carbone anomère à un radical, identique ou différent du groupement chromogène ou fluorigène du substrat, notamment alkyle linéaire ou ramifié substitué ou non, ayant de 1 à 20 atomes de carbone, de préférence 1 à 10 atomes de carbone, et notamment un radical méthyle ou isopropyle, ou alcoylène linéaire ou ramifié, substitué ou non, ayant de 1 à 20 atomes de carbone, de préférence 1 à 10 atomes de carbone, ou aromatique ou hétérocyclique, ayant de 6 à 10 atomes de carbone, substitué ou non.

Quand le composé hexosaminé est un analogue structural du substrat, ledit composé peut notamment être choisi parmi :
- les analogues structuraux du substrat dont la partie hexosaminée est identique à celle du substrat et le radical lié à l'oxygène porté par le carbone anomère est différent du groupement chromogène ou fluorigène du substrat, et
- les analogues structuraux du substrat dont la partie hexosaminée est différente de celle du substrat et le radical lié à l'oxygène porté par le carbone anomère est identique au groupement chromogène ou fluorigène du substrat.

Le rôle activateur du composé hexosaminé, quand il est un analogue structural du substrat, est également surprenant dans la mesure où l'on pourrait logiquement s'attendre dans ce cas à un phénomène d'inhibition de compétition entre le composé hexosaminé et le substrat fluorigène ou chromogène.

Grâce à l'invention, et comme démontré par le protocole expérimental établi ci-après, la réaction d'hydrolyse enzymatique peut être détectée facilement après 24 heures d'incubation.

On expose ci-après, de manière générale, la composition du milieu de culture, exprimée en g/l de milieu final.

Le milieu comprend une base nutritive nécessaire au développement des levures, et des activateurs de l'hexosaminidase selon la présente invention.

Les éléments constitutifs de la base nutritive comprennent :
- des peptones de 0,01 à 40 g/l, telles que la peptone de viande, le produit commercialisé par la Société BIOMERIEUX sous la marque bioSoyase ou analogue, ou encore un mélange de peptones ; de préférence, la peptone ou le mélange de peptones est présent dans le milieu à environ 6 g/l ± 0,5 g/l ;
- un extrait de levure de 0,01 à 40 g/l, de préférence environ 1,5 g/l, apportant des vitamines de croissance des levures ;
- une source de carbone, telle que le glucose, le glycérol, un acétate, un pyruvate, un lactate, l'arginine, un aminobutyrate, ou un mélange de ces composants, dans la proportion de 0 à 10 g/l ; la source de carbone est de préférence le glucose en une quantité de 1 g/l ;
- un tampon ajouté au milieu afin d'obtenir un pH favorable pour le développement de *Candida albicans*, compris entre 5 et 8,5 ; le tampon est choisi parmi les tampons phosphate, Tris, Hépès (acide N-2-hydroxyéthylpipérazine-N'-2-éthanesulfonique) et citrate dans la proportion de 2,5 à 100 mM ; de préférence, le tampon est un tampon phosphate 10 mM pour ajuster le pH du milieu à une valeur voisine de 7 ;
- Agar de 11 à 20 g/l, de préférence de 15 g/l.

Le substrat chromogène ou fluorigène peut être tout substrat chromogène ou fluorigène hydrolysable par une hexosaminidase, telle qu'une galactosaminidase, glucosaminidase ou mannosaminidase, pour libérer un produit coloré ou fluorescent. De préférence, le substrat est choisi parmi ceux présentant une forte coloration ou fluorescence avec peu de molécules, n'induisant pas de modifications du métabolisme des microorganismes, excepté pour l'activité enzymatique recherchée, ne nécessitant pas, pour la révélation du produit coloré ou fluorescent, dit marqueur, de réactif toxique pour les microorganismes, possédant une bonne solubilité dans l'eau, libérant après hydrolyse un marqueur qui ne diffuse pas ou très peu dans le milieu, afin que le signal reste limité au voisinage immédiat des colonies, et produisant un signal qui est visualisé sans forcément nécessiter l'emploi d'un appareil tel qu'une lampe UV ou un spectrophotomètre. Ces substrats sont de préférence choisis, pour les substrats chromogènes, parmi ceux comprenant un groupement chromophore tel qu'un indolyle substitué ou non, et notamment parmi le 5-bromo-4-chloro-3-indolyl-N-acétyl-β-D-glucosamine ou 5-bromo-4-chloro-3-indolyl-2-acétamido-2-désoxy-β-D-glucopyranoside et le 5-bromo-4-chloro-3-indolyl -N-acétyl-β-D-galactosamine ou 5-bromo-4-chloro-3-indolyl-2-acétamido-2-désoxy-β-D-galactopyranoside, de 20 à 600 µM, avantageusement le 5-bromo-4-chloro-3-indolyl-N-acétyl-β-D-glucosamine ou 5-bromo-4-chloro-3-indolyl-2-acétamido-2-désoxy-β-D-glucopyranoside à 200 µM, et pour les substrats fluorigènes, parmi la 4-méthylumbelliféryl-N-acétyl-β-D-galactosamine ou 4-méthylumbelliféryl-2-acétamido-2-désoxy-β-D-galactopyranoside, la 4-méthylumbelliféryl-N-acétyl-β-D-glucosamine ou 4-méthylumbelliféryl-2-acétamido-2-désoxy-β-D-glucopyranoside.

L'hexosamine activatrice de l'hexosaminidase est choisie dans le groupe des composés hexosaminés ou leur mélange. Elle est notamment choisie parmi les hexosamines simples telles que glucosamine, mannosamine et galactosamine, mais aussi parmi les hexosamines N-substituées, par exemple les N-acétyl-hexosamines ou 2-acétamido-2-désoxy-hexoses.

D'autres hexosamines sont selon l'invention des analogues structuraux du substrat, et comprennent une partie hexosaminée, identique ou différente de la partie hexosaminée du substrat, liées par l'oxygène porté par le carbone anomère à un radical, identique ou différent du groupement chromogène ou fluorigène du substrat, notamment alkyle linéaire ou ramifié substitué ou non, ayant de 1 à 20 atomes de carbone, et notamment un radical méthyle ou isopropyle, ou alcényle linéaire ou ramifié, substitué ou non, ayant de 2 à 20 atomes de carbone, ou à un radical aromatique hétérocyclique, ayant de 6 à 10 atomes de carbone, substitué ou non. Dans le cas où ledit radical alkyle ou alcényle est substitué, il l'est de préférence par un groupement thiol.

La quantité du composé hexosaminé est comprise entre 0,01 et 20 g/l. De préférence, l'activateur choisi est la N-acétyl-glucosamine ou 2-acétamido-2-désoxy-glucose en une quantité de 1 g/l.

D'autres effecteurs différents de l'activateur selon l'invention peuvent être ajoutés au milieu pour favoriser l'activité de l'hexosaminidase. Ces effecteurs sont choisis dans le groupe des cations métalliques bivalents tels que des sels de Mn²⁺, Mg²⁺, Ca²⁺ ou un mélange de ceux-ci, de 0 à 10 mM. De préférence, le composé cationique bivalent choisi est un sel de Mn²⁺ (1 mM).

Les agents de perméabilisation et/ou tensioactifs peuvent être ajoutés au milieu pour favoriser un bon contact enzyme/substrat. Les premiers sont choisis dans le groupe consistant en les sels biliaires tels que le désoxycholate de sodium, et les seconds sont choisis parmi les éthers de polyoxyéthylèneglycol et d'alkylphénol, commercialisés par exemple sous la marque Triton^{R}, les esters de sorbitanne et d'acides gras, commercialisés par exemple sous la marque Tween^{R}, dans la concentration de 0 à 5 g/l. De préférence, l'agent de perméabilisation est le désoxycholate de sodium dans une quantité de 0,1 g/l.

Un inhibiteur ou un mélange d'inhibiteurs des bactéries, permettant d'inhiber la croissance des bactéries gram+ et gram-, sans affecter celle des levures, et si possible des champignons, peut être ajouté au milieu. De préférence, les inhibiteurs de bactéries sont choisis dans le groupe des antibiotiques tels que gentamicine, chloramphénicol, pénicilline, streptomycine, cycloheximide, néomycine, tétracycline, oxytétracycline ou un mélange d'antibiotiques, et/ou parmi la tellurite, un molybdate et analogues, ou leurs mélanges. Avantageusement, on choisit le chloramphénicol (0,5 g/l), ou un mélange de gentamicine (0,1 g/l) et de chloramphénicol (0,05 g/l). Il est également possible d'inhiber la croissance des bactéries en diminuant le pH du milieu jusqu'à un pH acide.

Les caractéristiques et avantages de l'invention sont à présent exposés à l'appui des exemples 1 à 7 suivants.

### Exemple 1 :

On donne ci-après une composition du milieu préférentielle pour 1 litre :
- bioSoyase (BIOMERIEUX) 6,0 g
- extrait de levure (BIOMERIEUX) 1,5 g
- glucose (MERCK) 1,0 g
- N-acétyl-glucosamine (SIGMA) ou 2-acétamido-2-désoxy-glucose 1,0 g
- tampon phosphate (MERCK) 10,0 mmoles
- Mn²⁺ (MERCK) 1,0 mmole
- désoxycholate de sodium (MERCK) 0,1 g
- 5-bromo-4-chloro-3-indolyl-N-acétyl-β-D-glucosamine ou 5-bromo-4-chloro-3-indolyl-2-acétamido-2-désoxy-β-D-glucopyranoside (BIOSYNTH) 0,1 g
- agar (BIOMERIEUX) 15,0 g
- gentamicine 0,1 g
- chloramphénico 10,05 g
- pH 7,0

### Exemple 2 :

Des essais ont été réalisés pour tester les milieux de l'invention et les comparer à des milieux conventionnels.

Deux milieux ont été préparés selon les techniques habituelles. Le premier milieu ci-après désigné Milieu I contient tous les éléments de la base nutritive, ainsi qu'un substrat chromogène d'une hexosaminidase et un inhibiteur de bactéries.

La composition du Milieu I pour un litre de milieu final est la suivante :
- bioSoyase (BIOMERIEUX) 6,0 g
- extrait de levure (BIOMERIEUX) 1,5 g
- glucose (MERCK) 1,0 g
- tampon phosphate (MERCK) 10,0 mmoles
- 5-bromo-4-chloro-3-indolyl-N-acétyl-β-D-glucosamine ou 5-bromo-4-chloro-3-indolyl-2-acétamido-2-désoxy-β-D-glucopyranoside (BIOSYNTH) 0,1 g
- agar (BIOMERIEUX) 15,0 g
- gentamicine 0,1 g
- chloramphénico 10,05 g

Le pH du milieu a été ajusté aux environs de 7.

Le second milieu appelé Milieu II correspond au milieu selon l'invention et contient tous les éléments ci-dessus décrits pour le Milieu I, plus les activateurs de la présente invention, c'est-à-dire un composé hexosaminé, un agent de perméabilisation et des cations bivalents. La composition du Milieu II correspond à celle décrite dans l'exemple 1.

Sur ces deux milieux, 15 souches de levures ont été directement cultivées en boîte de Pétri. Les souches provenant de la collection de la Demanderesse, appartiennent aux espèces suivantes : *Candida albicans* : huit souches, *Candida glabrata* : trois souches, *Candida tropicalis* : quatre souches. Les boîtes ont été incubées à 37°C pendant 48 heures. Les colonies formées ont été examinées visuellement, respectivement après 24 et 48 heures d'incubation selon les interprétations suivantes :
- les colonies bleues correspondent à des souches *Candida albicans* produisant la N-acétyl-β-D-glucosaminidase ;
- les colonies blanches correspondent aux souches qui ne produisent pas l'enzyme précité et appartiennent donc à d'autres espèces de levures qui seront alors à identifier à l'aide des techniques habituelles.

Les résultats sont présentés dans le tableau I ci-après :

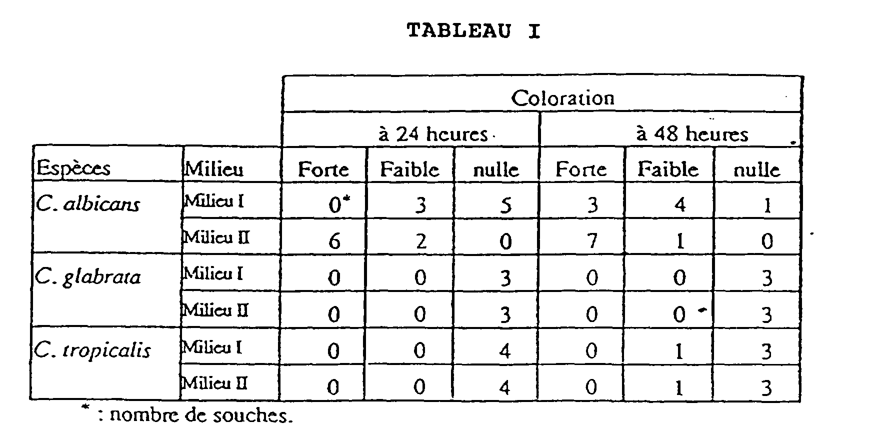

Comme cela ressort du tableau ci-dessus, l'apport de l'activateur de l'hexosaminidase permet une détection beaucoup plus précoce des souches de *Candida albicans*. En effet, toutes les souches sont colorées dès 24 heures sur le milieu selon l'invention, six sur huit l'étant fortement, alors que seulement trois le sont très faiblement sur le milieu conventionnel.

### Exemple 3 :

Les essais effectués dans l'Exemple 2, ont été reproduits dans cet exemple avec le même milieu conventionnel I et un milieu III selon l'invention dans lequel l'activateur du milieu II, c'est-à-dire la N-acétyl-glucosamine ou 2-acétamido-2-désoxy-glucose a été remplacée par un activateur analogue de structure du substrat, à savoir la méthyl-N-acétyl-β-D-glucosamine ou méthyl-2-acétamido-2-désoxy-β-D-glucopyranoside.

La concentration de l'activateur dans le milieu III est de 50 mg/l.

Les autres constituants du milieu III restent les mêmes que ceux du milieu II.

Les résultats des essais sont présentés dans le Tableau II suivant:

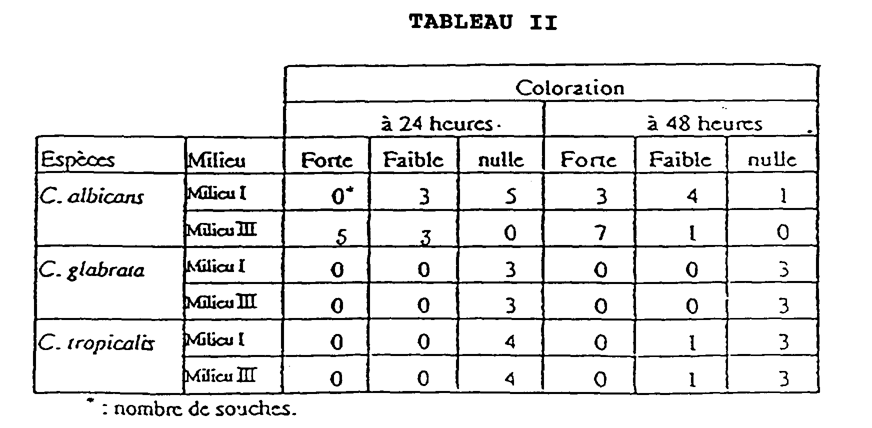

Comme dans l'exemple précédent, l'apport de l'activateur de l'hexosaminidase permet une détection beaucoup plus précoce des souches de *Candida albicans.* En effet, toutes les souches sont colorées dès 24 heures sur le milieu selon l'invention, cinq sur huit l'étant fortement, alors que seulement trois le sont très faiblement sur le milieu conventionnel.

### Exemple 4 :

42 souches de levures ou de bactéries ont été placées sur le milieu II de la présente invention. Ces souches proviennent toutes de la collection de la Demanderesse et se répartissent ainsi :
- 8: *Candida albicans*
- 3: *Candida (Torulopsis) glabrata*
- 2: *Candida guilliermondii*
- 2: *Candida kursei*
- 2: *Candida lusitaniae*
- 3: *Candida parapsilosis*
- 3: *Candida pseudotropicalis*
- *2*: *Candida rugosa*
- 2: *Candida stellatoidea*
- *4*: *Candida tropicalis*
- 3: *Cryptococcus neoformans*
- *2*: *Saccharomyces cerevisiae*
- *2*: *Trichosporon cutaneum*
- 2: *Serratia marcescens*
- 2: *Staphylococcus aureus*

Les boîtes de Pétri contenant le milieu II de la présente invention ont été ensemencées selon la procédure habituelle avec chacune des souches décrites ci-dessus. Les boîtes ont été incubées à 37°C pendant 48 heures. Les colonies formées ont été examinées visuellement, respectivement après 24 et 48 heures d'incubation.

Parmi les 42 isolements, seules les huit souches de *Candida albicans* ont donné des colonies bleues après 24 heures d'incubation, coloration qui caractérise *Candida albicans* sur le milieu de l'invention, six de ces souches étant très fortement colorées et deux un peu plus faiblement. Après 48 heures d'incubation, 7 des 8 souches de *Candida albicans* donnent des colonies fortement colorées en bleu, une seule ne l'étant que faiblement.

Parmi les autres espèces de levures, seules les souches de *Trichosporon cutaneum* ont une croissance nulle à 24 heures, et quasi nulle à 48 heures. Toutes les autres souches de levures donnent de petites colonies blanches à 24 heures, dont la taille s'accroît à 48 heures. Pour une souche de *Candida tropicalis* et les deux souches de *Candida stellatoidea,* on observe des colonies blanches après 24 heures d'incubation qui se colorent très faiblement en bleu après 48 heures d'incubation.

En fonction de l'intensité du signal obtenu avec le milieu de la présente invention pour la détection de *Candida albicans*, il est impossible de confondre la très faible coloration obtenue après 48 heures d'incubation avec ces deux autres espèces, de celle obtenue pour *Candida albicans*, même dans le cas de souches plus faiblement colorées de *Candida albicans*.

Par ailleurs, en ce qui concerne les souches de bactéries, c'est-à-dire *Serratia marcescens* et *Staphylococcus aureus*, aucune de ces souches ne produit de colonies, même après 48 heures d'incubation.

Il ressort donc de ces essais que le milieu de l'invention présente une très bonne sensibilité et spécificité pour *Candida albicans*, et permet de détecter et d'identifier directement et sans ambiguité les souches de *Candida albicans* qui sont les seules à produire des colonies bleues après 24 heures d'incubation.

### Exemple 5 :

Une comparaison a été effectuée entre le milieu commercialisé par MERCK sous la dénomination Fluoroplate Candida Agar et le milieu II selon l'invention.

Les 42 souches de l'exemple précédent ont été cultivées en boîtes de Pétri, respectivement sur chacun de ces milieux. Les boîtes ont été incubées à 37°C pendant 48 heures et les colonies formées ont été examinées visuellement après 24 et 48 heures d'incubation.

Sur le milieu de l'invention, les colonies exprimant l'activité d'une hexosaminidase sont bleues, alors que sur le milieu de MERCK, elles présentent une fluorescence bleue après observation sous lampe UV à 360 nm.

Les résultats sont présentés dans le tableau III ci-après.

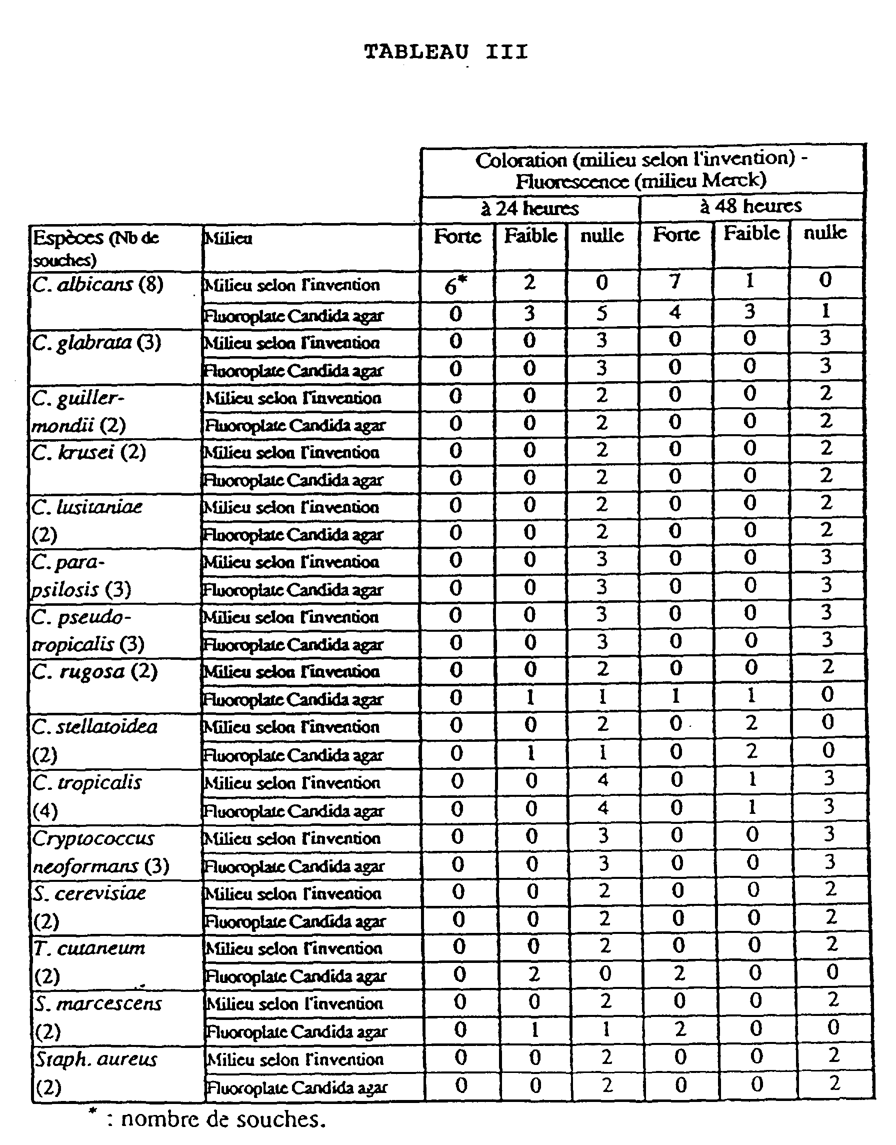

Comme cela ressort du tableau ci-dessus, les 8 souches de *Candida albicans* sont détectées dès 24 heures, dans la majorité des cas avec une très forte coloration avec le milieu de l'invention, alors que seulement trois souches produisent une fluorescence faible après 24 heures d'incubation sur le milieu commercialisé par la Société MERCK. De même, après 48 heures d'incubation, on constate que 7 souches de *Candida albicans* sont détectées avec une très forte coloration, contre seulement 4 avec le milieu Fluoroplate Candida Agar. Ces résultats démontrent l'excellente sensibilité du milieu de la présente invention, en comparaison avec celle du milieu antérieur.

Par ailleurs, le milieu de la présente invention possède une très bonne spécificité, puisqu'on n'observe pas de faux positif à 24 heures, alors que 4 souches autres que *Candida albicans* présentent une fluorescence avec le milieu Fluoroplate Candida Agar. Après 48 heures d'incubation, 3 souches n'appartenant pas à l'espèce *Candida albicans* paraissent très faiblement colorées avec le milieu de l'invention, contre 9 avec l'autre milieu dont certaines sont très fortement colorées.

Enfin, on observe une excellente sélectivité du milieu selon l'invention vis-à-vis des bactéries, ce qui évite de masquer la présence de levures sur des prélèvements fortement contaminés par des bactéries, qui peuvent également posséder une hexosaminidase.

Outre les excellentes sensibilité, spécificité et sélectivité obtenues avec le milieu de la présente invention, celui-ci présente encore l'avantage de permettre une lecture d'une coloration dès 24 heures, possible directement sur le milieu à l'oeil nu. De plus, la coloration intense obtenue avec le milieu de la présente invention limitée à la colonie, permet une détection des cultures plurimicrobiennes, alors que la fluorescence sur le milieu commercial diffuse dans toute la gélose.

### Exemple 6 :

On a effectué un essai pour déterminer le nombre minimal de colonies de *Candida albicans* pouvant être détectées, à partir d'un mélange de microorganismes, sur le milieu II de l'invention.

Cet essai a été effectué sur les 4 souches suivantes :
- *Candida albicans*, BIOMERIEUX référence 19
- *Candida (Torulopsis) glabrata,* BIOMERIEUX référence 154
- *Candida tropicalis*, BIOMERIEUX référence 47
- *Serratia marcescens*, BIOMERIEUX référence 106.

Ces souches ont été cultivées pendant 24 heures à 37°C sur gélose commercialisée sous la dénomination Sabouraud. Une suspension en sérum physiologique a été effectuée pour chaque souche, ajustée au néphélomètre de façon à obtenir environ 10⁸ germes/ml. Ces suspensions ont ensuite été diluées à 10⁴ fois. Pour la souche de *Candida albicans*, cette dernière suspension a été diluée 10 et 50 fois, permettant ainsi de disposer de trois dilutions de la suspension initiale : 10⁴ 10⁵ et 5.10⁵. Les différentes suspensions de la souche de *Candida albicans* ont ensuite été mélangées à volume égal avec celles des trois autres souches.

20 µl de chacun de ces mélanges ont été déposés sur des boîtes de Pétri contenant le milieu de l'invention et étalés à l'aide d'une pipette Pasteur stérile recourbée. Après absorption, la boîte est retournée et incubée pendant 24 heures à 37°C. Les colonies blanches et bleues sont comptées pour chaque boîte. Les résultats sont présentés dans le tableau IV ci-après.

**TABLEAU IV**

| | Espèces dans le mélange | | |
|---|---|---|---|
| Dilutions dans le mélange *C. albicans* / Autre souche | *C. albicans* + *C. glabrata* | *C. albicans* + *C. tropicalis* | *C. albicans* + *S. marcesens* |
| 10⁴/10⁴ | 123/152* | 93/107 | 113/0 |
| 10⁵/10⁴ | 9/119 | 12/122 | 11/0 |
| 5.10⁵/10⁴ | 3/135 | 1/97 | 2/0 |

| | | | |
|---|---|---|---|
| * : nombres de colonies bleues/nombre de colonies blanches. | | | |

D'après les résultats, il apparaît que sur le milieu II de l'invention, il est possible de mettre en évidence, sans aucune difficulté, les mélanges de souches de *Candida albicans*, c'est-à-dire les colonies bleues, avec celles d'autres espèces de levures, c'est-à-dire les colonies blanches. Cette détection est aussi aisée qu'il y ait une colonie parmi 100, ou qu'elles soient en grand nombre, la coloration des colonies de *Candida albicans* étant constante quel que soit leur nombre sur le milieu d'isolement. De plus, la présence de bactéries dans la suspension n'interfère en rien avec le développement et la coloration des souches de *Candida albicans* sur le milieu de l'invention.

### Exemple 7 :

Isolement de levures sur le milieu II de l'invention et utilisation de kits d'identification et de détermination des résistances aux antifongiques.

Quatre souches de levures provenant de la collection de la Demanderesse ont été isolées sur le milieu de l'invention, ainsi que sur le milieu Sabouraud Gentamicine Chloramphénicol (commercialisé par la Société BIOMERIEUX). Ces souches appartiennent aux espèces suivantes :
- 2: *Candida albicans*
- 1: *Candida tropicalis*
- 1: *Candida (Torulopsis) glabrata*

Pour chaque isolement, après 24 heures d'incubation à 37°C, des colonies ont servi à inoculer, selon les instructions du fabricant, une galerie d'identification des levures ID 32 C (nom commercial, Société BIOMERIEUX), une galerie ATB Fungus (antifongigramme) de la Société BIOMERIEUX (ATB marque enregistrée). Les colonies des souches de *Candida albicans* sur le milieu II de l'invention présentent une coloration bleue.

Les galeries sont lues respectivement à 24, 48 et 72 heures et les résultats reportés sur les fiches de lecture. Les résultats obtenus pour chaque souche isolée sur le milieu de l'invention sont identiques à ceux obtenus après isolement sur le milieu Sabouraud.

Les colonies de levures, appartenant ou non à l'espèce *Candida albicans,* isolées sur le milieu II de l'invention peuvent servir pour l'étude de leur résistance aux antifongiques, ou pour inoculer les kits d'identification des levures.

## Revendications

1. Milieu de culture et de détection sélective de levures de l'espèce *Candida albicans,* comprenant une base nutritive métabolisée par lesdites levures, un substrat comprenant une partie hexosaminée liée par l'oxygène porté par le carbone anomère à un groupement chromogène ou fluorigène, ledit sustrat étant susceptible d'être hydrolysé par un enzyme du groupe des hexosaminidases pour libérer ladite partie hexosaminée et un produit coloré ou fluorescent, caractérisé en ce que le milieu comprend en outre au moins un composé hexosaminé qui est différent du substrat.

2. Milieu selon la revendication 1, caractérisé en ce que le composé hexosaminé appartient au groupe des hexosamines simples identiques à la partie hexosaminée du substrat.

3. Milieu selon la revendication 1, caractérisé en ce que le composé hexosaminé appartient au groupe des hexosamines simples différentes de la partie hexosaminée du substrat.

4. Milieu selon la revendication 1, 2 ou 3, caractérisé en ce que le composé hexosaminé est une hexosamine choisie parmi la glucosamine, la galactosamine et la mannosamine.

5. Milieu selon la revendication 1, caractérisé en ce que le composé hexosaminé est une hexosamine N-substituée.

6. Milieu selon la revendication 5, caractérisé en ce que l'hexosamine N-substituée est une N-acétyl-hexosamine.

7. Milieu selon la revendication 1, caractérisé en ce que le composé hexosaminé appartient au groupe des hexosamines complexes.

8. Milieu selon la revendication 7, caractérisé en ce que le composé hexosaminé appartient au groupe des hexosamines qui sont des analogues structuraux du substrat, comprenant une partie hexosaminée, identique ou différente de la partie hexosaminée du substrat, liée par l'oxygène porté par le carbone anomère à un radical, identique ou différent du groupement chromogène ou fluorigène du substrat, notamment alkyle linéaire ou ramifié substitué ou non, ayant de 1 à 20 atomes de carbone, et notamment un radical méthyle ou isopropyle, ou alcényle linéaire ou ramifié, substitué ou non, ayant de 2 à 20 atomes de carbone, ou aromatique ou hétérocyclique ayant de 6 à 10 atomes de carbone, substitué ou non.

9. Milieu selon la revendication 8, caractérisé en ce que la partie hexosaminée est N-substituée et plus particulièrement N-acétylée.

10. Milieu selon la revendication 8, caractérisé en ce que la partie hexosaminée du composé hexosaminé est identique à la partie hexosaminée du substrat, et le radical lié à l'oxygène porté par le carbone anomère est différent du groupement chromogène ou fluorigène du substrat.

11. Milieu selon la revendication 8, caractérisé en ce que la partie hexosaminée du composé hexosaminé est différente de la partie hexosaminée du substrat, et le groupement chromogène ou fluorigène du composé hexosaminé est identique au groupement chromogène ou fluorigène du substrat.

12. Milieu selon la revendication 1, caractérisé en ce que le composé hexosaminé est présent à raison d'une quantité comprise entre 0,01 g et 20 g pour un litre.

13. Milieu selon les revendications 6 et 12, caractérisé en ce que le milieu d'identification comprend une N-acétyl-glucosamine, en une quantité de 1 g pour 1 litre de milieu.

14. Milieu selon la revendication 1, caractérisé en ce qu'il comprend au moins un effecteur activant l'hexosaminidase, différent dudit activateur et choisi parmi les cations métalliques bivalents.

15. Milieu selon la revendication 14, caractérisé en ce que les cations métalliques bivalents sont des sels de Mn²⁺, Mg²⁺, Ca²⁺, et la concentration molaire de l'activateur dans le milieu est comprise entre 0 et 10 mM.

16. Milieu selon la revendication 15, caractérisé en ce que l'activateur est un sel de Mn²⁺, et sa concentration molaire dans le milieu est de 1 mM.

17. Milieu selon la revendication 1, caractérisé en ce qu'il comprend au moins un agent de perméabilisation de la paroi cellulaire de la levure choisi parmi les sels biliaires tels que le désoxycholate de sodium.

18. Milieu selon la revendication 1, caractérisé en ce qu'il comprend au moins un agent tensioactif.

19. Milieu selon la revendication 18, caractérisé en ce que le tensioactif est choisi parmi les éthers de polyoxyéthylèneglycol et d'alkylphénol, et les esters de sorbitanne et d'acides gras.

20. Milieu selon l'une des revendications 17, 18 et 19, caractérisé en ce que l'agent de perméabilisation et/ou le tensioactif sont présents dans le milieu d'identification en une quantité comprise entre 0 et 5 g par litre de milieu.

21. Milieu selon la revendication 1, caractérisé en ce qu'il comprend un inhibiteur de croissance des bactéries.

22. Milieu selon la revendication 21, caractérisé en ce que l'inhibiteur de croissance des bactéries est constitué par au moins un antibiotique choisi parmi la gentamicine, le chloramphénicol, la pénicilline, la streptomycine, le cycloheximide, la néomycine, la tétracycline et l'oxytétracycline.

23. Milieu selon la revendication 22, caractérisé en ce que l'inhibiteur de croissance des bactéries est le chloramphénicol à raison de 0,5 g par litre de milieu, ou un mélange de gentamicine et de chloramphénicol, à raison de respectivement 0,1 g et 0,05 g par litre de milieu.

24. Milieu selon la revendication 21, caractérisé en ce que l'inhibiteur de croissance des bactéries est choisi parmi la tellurite et un molybdate.

25. Milieu selon la revendication 1, caractérisé en ce que le substrat chromogène ou fluorigène est choisi parmi la 4-méthylumbelliféryl-N-acétyl-β-D-galactosamine, la 4-méthylumbelliféryl-N-acétyl-β-D-glucosamine, la 5-bromo-4-chloro-3-indolyl-N-acétyl-β-D-glucosamine et la 5-bromo-4-chloro-3-indolyl-N-acétyl-β-D-galactosamine, et sa concentration molaire dans le milieu est comprise entre 20 et 600 µM.

26. Milieu selon la revendication 25, caractérisé en ce que le substrat chromogène est la 5-bromo-4-chloro-3-indolyl-N-acétyl-β-D-glucosamine et sa concentration molaire dans le milieu est de 200 µM.

27. Milieu selon la revendication 1, caractérisé en ce que la base nutritive comprend une peptone en quantité comprise entre 0,01 et 40 g par litre de milieu, un extrait de levure en quantité comprise entre 0,01 et 40 g par litre de milieu, et une source de carbone choisie parmi le glucose, le glycérol, les acétates, les pyruvates, les lactates, l'arginine, les amino-butyrates, la quantité de ladite source de carbone étant comprise entre 0 et 10 g par litre de milieu.

28. Milieu selon la revendication 1, caractérisé en ce qu'il comprend un tampon pour ajuster le milieu à un pH compris entre 5 et 8,5.

29. Milieu selon la revendication 1, caractérisé en ce qu'il comprend de l'agar dans la proportion de 11 à 20 g/l, par exemple de 15 g/l.

30. Dispositif de caractérisation microbiologique comprenant un conditionnement d'un milieu selon l'une quelconque des revendications 1 à 29, agencé pour permettre et l'inoculation d'un échantillon à tester sur le milieu, et son incubation.

31. Procédé d'analyse microbiologique pour détecter sélectivement les levures de l'espèce *Candida albicans* dans un échantillon biologique, caractérisé en ce qu'il comprend les étapes suivantes :
- on dipose d'un milieu d'identification selon l'une quelconque des revendications 1 à 29,
- on met directement l'échantillon à analyser au contact du milieu d'identification,
- on incube l'échantillon en contact avec le milieu d'identification,
- on observe l'absence ou la présence du produit coloré ou fluorescent résultant de l'hydrolyse du substrat par une hexosaminidase.

## Patentansprüche

1. Kultur- und selektives Nachweismedium für Hefen der Gattung *Candida albicans,* umfassend eine Nährbasis, die von den Hefen metabolisiert wird, ein Substrat, umfassend einen Hexosaminanteil, der über den Sauerstoff am anomeren Kohlenstoff an eine chromogene oder fluorogene Gruppe gebunden ist, wobei das Substrat durch ein Enzym aus der Gruppe der Hexosaminidasen hydrolisiert werden kann um den Hexosaminanteil und ein gefärbtes oder fluoreszierendes Produkt freizusetzen, dadurch gekennzeichnet, daß das Medium weiterhin mindestens eine vom Substrat unterschiedliche Hexosaminverbindung umfaßt.

2. Medium nach Anspruch 1, dadurch gekennzeichnet, daß die Hexosaminverbindung der Gruppe der einfachen Hexosamine, die zum Hexosaminanteil des Substrats identisch sind, angehört.

3. Medium nach Anspruch 1, dadurch gekennzeichnet, daß die Hexosaminverbindung der Gruppe der einfachen Hexosamine, die vom Hexosaminanteil des Substrats unterschiedlich sind, angehört.

4. Medium nach Anspruch 1, 2 oder 3, dadurch gekennzeichnet, daß die Hexosaminverbindung ein Hexosamin, ausgewählt aus Glucosamin, Galactosamin, und Mannosamin ist.

5. Medium nach Anspruch 1, dadurch gekennzeichnet, daß die Hexosaminverbindung ein N-substituiertes Hexosamin ist.

6. Medium nach Anspruch 5, dadurch gekennzeichnet, daß das N-substituierte Hexosamin ein N-Acetyl-hexosamin ist.

7. Medium nach Anspruch 1, dadurch gekennzeichnet, daß die Hexosaminverbindung der Gruppe der komplexen Hexosamine angehört.

8. Medium nach Anspruch 7, dadurch gekennzeichnet, daß die Hexosaminverbindung der Gruppe der zum Substrat strukturanalogen Hexosamine angehört, umfassend einen zum Hexosaminanteil des Substrats identischen oder unterschiedlichen Hexosaminanteil, der über den Sauerstoff am anomeren Kohlenstoff an einen zu der chromogenen oder fluorogenen Gruppe des Substrats identischen oder unterschiedlichen Rest gebunden ist, insbesondere einen substituierten oder nicht-substituierten geradkettigen oder verzweigten Alkylrest mit 1 bis 20 Kohlenstoffatomen und insbesondere einen Methyl- oder Isopropylrest, oder einen substituierten oder nicht-substituierten geradkettigen oder verzweigten Alkenylrest mit 2 bis 20 Kohlenstoffatomen, oder einen substituierten oder nicht-substituierten aromatischen oder heterozyklischen Rest mit 6 bis 10 Kohlenstoffatomen.

9. Medium nach Anspruch 8, dadurch gekennzeichnet, daß der Hexosaminanteil N-substituiert und insbesonere N-acetyliert ist.

10. Medium nach Anspruch 8, dadurch gekennzeichnet, daß der Hexosaminanteil der Hexosaminverbindung zu dem Hexosaminanteil des Substrats identisch ist und der an den Sauerstoff am anomeren Kohlenstoff gebundene Rest von der chromogenen oder fluorogenen Gruppe des Substrats unterschiedlich ist.

11. Medium nach Anspruch 8, dadurch gekennzeichnet, daß der Hexosaminanteil der Hexosaminverbindung von dem Hexosaminanteil des Substrats unterschiedlich ist und die chromogene oder fluorogene Gruppe der Hexosaminverbindung zu der chromogenen oder fluorogenen Gruppe des Substrats identisch ist.

12. Medium nach Anspruch 1, dadurch gekennzeichnet, daß die Hexosaminverbindung in einer Menge zwischen 0,01 g und 20 g pro Liter vorhanden ist.

13. Medium nach den Ansprüchen 6 und 12, dadurch gekenzeichnet, daß das Identifizierungsmedium ein N-Acetyl-glucosamin in einer Menge von 1 g pro Liter Medium umfaßt.

14. Medium nach Anspruch 1, dadurch gekennzeichnet, daß es mindestens einen die Hexosaminidase aktivierenden Effektor umfaßt, der von dem Aktivator unterschiedlich ist und aus den zweiwertigen Metall-Kationen ausgewählt ist.

15. Medium nach Anspruch 14, dadurch gekennzeichnet, daß die zweiwertigen Metall-Kationen Salze von Mn²⁺, Mg^{2+,} Ca²⁺ sind und die molare Konzentration des Aktivators in dem Medium zwischen 0 und 10 mM beträgt.

16. Medium nach Anspruch 15, dadurch gekennzeichnet, daß der Aktivator ein Mn²⁺-Salz ist und seine molare Konzentration in dem Medium 1 mM beträgt.

17. Medium nach Anspruch 1, dadurch gekennzeichnet, daß es mindestens ein die Zellwand der Hefe permeabilisierendes Mittel, ausgewählt aus den Gallensalzen wie etwa Natriumdesoxycholat, umfaßt.

18. Medium nach Anspruch 1, dadurch gekennzeichnet, daß es mindestens ein oberflächenaktives Mittel umfaßt.

19. Medium nach Anspruch 18, dadurch gekennzeichnet, daß das oberflächenaktive Mittel ausgewählt ist aus den Polyoxyethylenglykol- und Alkylphenolethern und den Sorbitan- und Fettsäureestern.

20. Medium nach einem der Ansprüche 17, 18 und 19, dadurch gekennzeichnet, daß das permeabilisierende Mittel und/oder das oberflächenaktive Mittel in dem Identifizierungsmedium in einer Menge zwischen 0 und 5 g pro Liter Medium vorhanden ist.

21. Medium nach Anspruch 1, dadurch gekennzeichnet, daß es einen Bakterienwachstumsinhibitor umfaßt.

22. Medium nach Anspruch 21, dadurch gekennzeichnet, daß der Bakterienwachstumsinhibitor aus mindestens einem Antibiotikum, ausgewählt aus Gentamicin, Chloramphenicol, Penicillin, Streptomycin, Cycloheximid, Neomycin, Tetracyclin und Oxytetracyclin besteht.

23. Medium nach Anspruch 22, dadurch gekennzeichnet, daß der Bakterienwachstumsinhibitor Chloramphenicol zu 0,5 g pro Liter Medium oder ein Gemisch von Gentamicin und Chloramphenicol zu 0,1 g bzw. 0,05 g pro Liter Medium ist.

24. Medium nach Anspruch 21, dadurch gekennzeichnet, daß der Bakterienwachstumsinhibitor aus Tellurit und einem Molybdat ausgewählt ist.

25. Medium nach Anspruch 1, dadurch gekennzeichnet, daß das chromogene oder fluorogene Substrat ausgewählt ist aus 4-Methylumbelliferyl-N-acetyl-β-D-galactosamin, 4-Methylumbelliferyl-N-acetyl-β-D-glucosamin, 5-Brom-4-chlor-3-indolyl-N-acetyl-β-D-glucosamin und 5-Brom-4-chlor-3-indolyl-N-acetyl-β-D-galactosamin und seine molare Konzentration in dem Medium zwischen 20 und 600 µM beträgt.

26. Medium nach Anspruch 25, dadurch gekennzeichnet, daß das chromogene Substrat 5-Brom-4-chlor-3-indolyl-N-acetyl-β-D-glucosamin ist und seine molare Konzentration in dem Medium 200 µM beträgt.

27. Medium nach Anspruch 1, dadurch gekennzeichnet, daß die Nährbasis ein Pepton in einer Menge zwischen 0,01 und 40 g pro Liter Medium, einen Hefeextrakt in einer Menge zwischen 0,01 und 40 g pro Liter Medium und eine Kohlenstoffquelle ausgewählt aus Glucose, Glyzerin, Acetaten, Pyruvaten, Lactaten, Arginin, Aminobutyraten umfaßt, wobei die Menge der Kohlenstoffquelle zwischen 0 und 10 g pro Liter Medium beträgt.

28. Medium nach Anspruch 1, dadurch gekennzeichnet, daß es einen Puffer umfaßt um das Medium auf einen pH-Wert zwischen 5 und-8,5 einzustellen.

29. Medium nach Anspruch 1, dadurch gekennzeichnet, daß es Agar im Anteil von 11 bis 20 g/l, beispielsweise 15 g/l umfaßt.

30. Mikrobiologische Charakterisierungsvorrichtung, umfassend eine Aufmachung eines Mediums nach einem der Ansprüche 1 bis 29, welche eingerichtet ist um das Animpfen einer zu untersuchenden Probe auf dem Medium und deren Inkubation zu gestatten.

31. Mikrobiologisches Analyseverfahren zum selektiven Nachweis von Hefen der Gattung *Candida albicans* in einer biologischen Probe, dadurch gekennzeichnet, daß es die folgenden Schritte umfaßt:
- Bereitstellen eines Identifizierungsmediums nach einem der Ansprüche 1 bis 29,
- direktes Inkontaktbringen der zu untersuchenden Probe mit dem Identifizierungsmedium,
- Inkubieren der Probe in Kontakt mit dem Identifizierungsmedium,
- Beobachten der Abwesenheit oder Gegenwart des aus der Hydrolyse des Substrats durch eine Hexosaminidase resultierenden gefärbten oder fluoreszierenden Produkts.

## Claims

1. Culture and detection medium selective for yeasts of the species *Candida albicans,* comprising a nutrient base metabolized by the said yeasts, a substrate comprising a hexosamine-containing portion attached by the oxygen carried by the anomeric carbon to a chromogenic or fluorigenic group, the said substrate being capable of being hydrolysed by an enzyme of the group comprising hexosaminidases to release the said hexosamine-containing portion and a coloured or fluorescent product, characterized in that the medium additionally comprises at least one hexosamine-containing compound which is different from the substrate.

2. Medium according to Claim 1, characterized in that the hexosamine-containing compound belongs to the group comprising simple hexosamines identical to the hexosamine-containing portion of the substrate.

3. Medium according to Claim 1, characterized in that the hexosamine-containing compound belongs to the group comprising simple hexosamines different from the hexosamine-containing portion of the substrate.

4. Medium according to Claim 1, 2 or 3, characterized in that the hexosamine-containing compound is a hexosamine chosen from glucosamine, galactosamine and mannosamine.

5. Medium according to Claim 1, characterized in that the hexosamine-containing compound is an N-substituted hexosamine.

6. Medium according to Claim 5, characterized in that the N-substituted hexosamine is an N-acetylhexosamine.

7. Medium according to Claim 1, characterized in that the hexosamine-containing compound belongs to the group comprising complex hexosamines.

8. Medium according to Claim 7, characterized in that the hexosamine-containing compound belongs to the group comprising hexosamines which are structural analogues of the substrate, comprising a hexosamine-containing portion, identical to or different from the hexosamine-containing portion of the substrate, attached by the oxygen carried by the anomeric carbon to a radical which is identical to or different from the chromogenic or fluorigenic group of the substrate, especially a substituted or unsubstituted, linear or branched alkyl having 1 to 20 carbon atoms, and especially a methyl radical or isopropyl radical, or a substituted or unsubstituted, linear or branched alkenyl radical having 2 to 20 carbon atoms, or a substituted or unsubstituted aromatic or heterocyclic radical having 6 to 10 carbon atoms.

9. Medium according to Claim 8, characterized in that the hexosamine-containing portion is N-substituted and more particularly N-acetylated.

10. Medium according to Claim 8, characterized in that the hexosamine-containing portion of the hexosamine-containing compound is identical to the hexosamine-containing portion of the substrate, and the radical attached to the oxygen carried by the anomeric carbon is different from the chromogenic or fluorigenic group of the substrate.

11. Medium according to Claim 8, characterized in that the hexosamine-containing portion of the hexosamine-containing compound is different from the hexosamine-containing portion of the substrate, and the chromogenic or fluorigenic group of the hexosamine-containing compound is identical to the chromogenic or fluorigenic group of the substrate.

12. Medium according to Claim 1, characterized in that the hexosamine-containing compound is present in an amount of between 0.01 g and 20 g per one litre.

13. Medium according to Claims 6 and 12, characterized in that the identification medium comprises an N-acetylglucosamine in an amount of 1 g per 1 litre of medium.

14. Medium according to Claim 1, characterized in that it comprises at least one effector activating hexosaminidase, different from the said activator and chosen from divalent metal cations.

15. Medium according to Claim 14, characterized in that the divalent metal cations are Mn²⁺, Mg²⁺ or Ca²⁺ salts, and the molar concentration of the activator in the medium is between 0 and 10 mM.

16. Medium according to Claim 15, characterized in that the activator is an Mn²⁺ salt, and its molar concentration in the medium is 1 mM.

17. Medium according to Claim 1, characterized in that it comprises at least one agent which enhances the permeability of the yeast cell wall, chosen from the bile salts, such as sodium deoxycholate.

18. Medium according to Claim 1, characterized in that it comprises at least one surface-active agent.

19. Medium according to Claim 18, characterized in that the surface-active agent chosen from the polyoxyethylene glycol and alkylphenol ethers, and sorbitan and fatty acid esters.

20. Medium according to one of Claims 17, 18 and 19, characterized in that the permeability-enhancing and/or surface-active agents are present in the identification medium in an amount of between 0 and 5 g per litre of medium.

21. Medium according to Claim 1, characterized in that it comprises a bacteria growth inhibitor.

22. Medium according to Claim 21, characterized in that the bacteria growth inhibitor consists of at least one antibiotic chosen from gentamicin, chloramphenicol, penicillin, streptomycin, cycloheximide, neomycin, tetracycline and oxytetracycline.

23. Medium according to Claim 22, characterized in that the bacteria growth inhibitor is chloramphenicol in an amount of 0.5 g per litre of medium, or a mixture of gentamicin and chloramphenicol, in an amount of 0.1 g and 0.05 g per litre of medium respectively.

24. Medium according to Claim 21, characterized in that the bacteria growth inhibitor is chosen from tellurite and a molybdate.

25. Medium according to Claim 1, characterized in that the chromogenic or fluorigenic substrate is chosen from 4-methylumbelliferyl-N-acetyl-β-D-galactosamine, 4-methylumbelliferyl-N-acetyl-β-D-glucosamine, 5-bromo-4-chloro-3-indolyl-N-acetyl-β-D-glucosamine and 5-bromo-4-chloro-3-indolyl-N-acetyl-β-D-galactosamine, and its molar concentration in the medium is between 20 and 600 µM.

26. Medium according to Claim 25, characterized in that the chromogenic substrate is 5-bromo-4-chloro-3-indolyl-N-acetyl-β-D-glucosamine and its molar concentration in the medium is 200 µM.

27. Medium according to Claim 1, characterized in that the nutrient base comprises a peptone in an amount of between 0.01 and 40 g per litre of medium, a yeast extract in an amount of between 0.01 and 40 g per litre of medium and a carbon source chosen from glucose, glycerol, acetates, pyruvates, lactates, arginine or aminobutyrates, the amount of the said carbon source being between 0 and 10 g per litre of medium.

28. Medium according to Claim 1, characterized in that it comprises a buffer for adjusting the medium to a pH of between 5 and 8.5.

29. Medium according to Claim 1, characterized in that it comprises agar in a proportion from 11 to 20 g/l, for example 15 g/l.

30. Device for microbiological characterization comprising a packaging of a medium according to any one of Claims 1 to 29, designed to allow both the inoculation of a test sample into the medium and its incubation.

31. Method of microbiological analysis for selectively detecting yeasts of the species *Candida albicans* in a biological sample, characterized in that it comprises the following steps:
- an identification medium according to any one of Claims 1 to 29 is obtained,
- the sample to be analyzed is placed directly into contact with the identification medium,
- the sample in contact with the identification medium is incubated,
- the absence or the presence of the coloured or fluorescent product resulting from the hydrolysis of the substrate by a hexosaminidase is observed.
